Europäisches Patentamt

European Patent Office

Office européen des brevets

(11) Veröffentlichungsnummer: **0 405 282 A1**

(12) **EUROPÄISCHE PATENTANMELDUNG**

(21) Anmeldenummer: 90111457.9

(22) Anmeldetag: 18.06.90

(51) Int. Cl.5: **A61B 17/22**

(30) Priorität: 30.06.89 EP 89111988
30.06.89 DE 8908037 U

(43) Veröffentlichungstag der Anmeldung:
02.01.91 Patentblatt 91/01

(84) Benannte Vertragsstaaten:
**DE FR GB NL**

(71) Anmelder: Siemens Aktiengesellschaft
Wittelsbacherplatz 2
**D-8000 München 2(DE)**

(72) Erfinder: **Goldhorn, Klaus**
**Ganghoferweg 2**
**D-8520 Erlangen(DE)**
Erfinder: **Seubert, Hans-Peter**
**Am Kübellohberg 5**
**D-8551 Heroldsbach(DE)**

(54) Gerät zur Behandlung eines Lebewesens mit fukussierten Stosswellen.

(57) Die Erfindung betrifft ein Gerät zur Behandlung eines Lebewesens mit fokussierten Stoßwellen, das als Gerätekomponenten eine Patientenliege (1), eine Ortungseinrichtung (29) und eine Stoßwellenquelle (11) aufweist, mittels derer in einem mit einem mittels der Ortungseinrichtung georteten therapeutisch relevanten Bereich (26) zusammen fallenden Fokusbereich (F) zusammenlaufende Stoßwellen erzeugbar sind. Dabei ist vorgesehen, daß eine gemeinsame, im wesentlichen horizontal verlaufende Schwenkachse (GS) vorgesehen ist, um die wahlweise die Gerätekomponenten (1, 11, 29) unabhängig voneinander oder wenigstens zwei der Gerätekomponenten (1, 11, 29) synchron schwenkbar sind.

FIG 8

# GERÄT ZUR BEHANDLUNG EINES LEBEWESENS MIT FOKUSSIERTEN STOSSWELLEN

Die Erfindung betrifft ein Gerät zur Behandlung eines Lebewesens mit fokussierten Stoßwellen, welches als Gerätekomponenten eine Patientenliege und eine Stoßwellenquelle aufweist, die relativ zu der Patientenliege verstellbar ist, die Mittel zur akustischen Koppelung mit dem Körper des Lebewesens aufweist und mittels derer in einem auf der akustischen Achse der Stoßwellenquelle liegenden Fokusbereich zusammenlaufende Stoßwellen erzeugbar sind.

Solche Geräte werden zur Zeit hauptsächlich zur berührungslosen Zertrümmerung von im Körperinneren befindlichen Konkrementen, z.B. Nieren- oder Gallenblasensteinen eingesetzt. Ihre Verwendung wurde aber auch bereits im Zusammenhang mit der Heilung bzw. Behandlung anderer Leiden vorgeschlagen, z.B. bei der Tumortherapie oder der Behandlung von Gefäßerkrankungen.

Ein Gerät der eingangs genannten Art, das zur berührungslosen Zertrümmerung von im Körper eines Lebewesens befindlichen Konkrementen dient, ist in der Druckschrift der Fa. Dornier Medizintechnik "Dornier Lithotripter MPL 9000" beschrieben. Dabei wird mittels einer Ortungseinrichtung die Position des zu zertrümmernden Konkrementes bestimmt und anschließend durch Verstellen der Stoßwellenquelle und der Patientenliege relativ zueinander das Konkrement in den Fokusbereich der Stoßwellen gebracht. Unter der Einwirkung einer Folge von mittels der Stoßwellenquelle erzeugter Stoßwellen zerfällt das Konkrement in feine Bruchstücke, die auf natürlichem Wege abgehen können. Um den Patienten in der erforderlichen Weise ausrichten zu können, ist die Patientenliege u.a. um eine horizontale, quer zur Längsachse der Patientenliege verlaufende Achse schwenkbar. Außerdem ist die Stoßwellenquelle in bezug auf den Fokusbereich der Stoßwellen isozentrisch verstellbar, d.h. die Stoßwellenquelle ist auf einem Kugelschalenabschnitt verstellbar, dessen Mittelpunkt im Fokusbereich liegt.

Insbesondere bei der Zertrümmerung von Gallenblasensteinen tritt das Problem auf, daß die Steine unter der Einwirkung der Stoßwellen laufend ihre Position ändern. Wenn dies der Fall ist, muß versucht werden, den Patienten durch Verstellen der Patientenliege in eine derart geneigte Position zu bringen, daß die Steine unter der Einwirkung der Schwerkraft eine stabile Lage einnehmen, die sie auch unter der Einwirkung der Stoßwellen beibehalten. Dies kann bei dem bekannten Gerät an sich durch Schwenken der Patientenliege um die horizontale Achse erreicht werden, indem der Patient den Erfordernissen des jeweiligen Therapiefalles entsprechend entweder in eine Kopf-oder Fußtieflage gebracht wird. Allerdings treten dabei solche Verlagerungen von Patientenliege und Stoßwellenquelle relativ zueinander auf, daß sich dann die Steine nicht mehr im Fokusbereich der Stoßwellen befinden. Es ist also erforderlich, die Steine vor der Weiterführung der Behandlung erneut zu orten und den Patienten durch Verstellen der Patientenliege wieder so auszurichten, daß sich die Steine im Fokusbereich befinden.

Der Erfindung liegt die Aufgabe zugrunde, ein Gerät der eingangs genannten Art so auszubilden, daß beim Schwenken der Patientenliege die Ausrichtung der Stoßwellenquelle auf das Zielgebiet erhalten bleibt.

Nach der Erfindung wird diese Aufgabe dadurch gelöst, daß eine gemeinsame Schwenkachse vorgesehen ist, um die die Gerätekomponenten wahlweise unabhängig voneinander oder synchron schwenkbar sind. Unter anderem infolge des Umstandes, daß die Patientenliege und die Stoßwellenquelle somit unabhängig voneinander schwenkbar sind, besteht also zunächst die Möglichkeit, die Stoßwellenquelle und die Patientenliege mit einem darauf liegenden Patienten derart relativ zueinander so zu verstellen, daß der Fokusbereich der an den Körper des Lebewesens akustisch angekoppelten Stoßwellenquelle in einem therapeutisch relevanten Bereich als Zielgebiet im Körper des Patienten liegt. Im Anschluß daran besteht infolge des Umstandes, daß die Patientenliege und die Stoßwellenquelle um die gemeinsame Schwenkachse auch synchron schwenkbar sind, die Möglichkeit, während der Behandlung die Patientenliege mit dem darauf liegenden Patienten gemeinsam mit der Stoßwellenquelle um die gemeinsame Schwenkachse in der jeweils erforderlichen Weise zu schwenken, ohne daß sich der Fokusbereich der Stoßwellen aus dem einmal georteten Zielgebiet verlagert. Eine erneute Ausrichtung der Stoßwellenquelle und der Patientenliege mit dem darauf liegenden Patienten relativ zueinander nach dem Schwenkvorgang ist also in der Regel vermieden.

Es sind außerdem Geräte der eingangs genannten Art bekannt, die als zusätzliche Gerätekomponente eine relativ zu den anderen Gerätekomponenten verstellbare Ortungseinrichtung aufweisen, die zur Ortung eines therapeutisch relevanten Bereiches, z.B. eines Konkrementes, dient, auf den als Zielgebiet der Stoßwellengenerator ausgerichtet wird. Ein Gerät dieser Art ist in der Druckschrift der Fa. Philips, C.H.F. Müller, Medizinisch-Technische Systeme, "MFL 5000 - Der urologische Arbeitsplatz für Diagnose und Therapie" beschrieben. Dabei wird mittels einer Röntgen-Ortungseinrichtung die Position des therapeutisch relevanten Bereiches, also des zu zertrümmernden Konkrementes, bestimmt und anschließend das Konkrement durch Verstellen der Patientenliege relativ zu der Stoßwellenquelle und zu der Ortungseinrichtung in den Fokusbereich der Stoßwellen gebracht. Unter der Einwirkung einer Folge von mittels der Stoßwellenquelle erzeugter Stoßwellen zerfällt das Konkrement in feine Bruchstücke, die auf natürlichem Wege ab gehen können. Im Falle des bekannten Gerätes weist die Röntgen-Ortungseinrichtung einen Röntgenstrahler und einen als Röntgenbildverstärker ausgeführten Strahlenempfänger auf, die an den entgegengesetzten Enden eines U-förmigen Bogens angebracht sind, der seinerseits um eine horizontale Achse schwenkbar an einem Ständer angebracht ist. Der Schnittpunkt des Zentralstrahles der Röntgen Ortungseinrichtung mit der horizontalen Achse entspricht der Lage des Fokusbereiches der Stoßwellen. Die Stoßwellenquelle ist an einer zur vertikalen geneigten Achse, die durch den Schnittpunkt des Zentralstrahles mit der horizontalen Achse verläuft, derart schwenkbar angebracht, daß ihr Zentralstrahl stets durch den besagten Schnittpunkt verläuft und ihr Fokusbereich die erwähnte Position einnimmt. Um den Patienten in der erforderlichen Weise ausrichten zu können, ist die Patientenliege u.a. um eine weitere horizontale, quer zur Längsachse der Patientenliege verlaufende Achse schwenkbar.

Wie bereits erläutert, muß insbesondere bei der Zertrümmerung von Gallenblasensteinen unter Umständen versucht werden, den Patienten durch Verstellen der Patientenliege in eine derart geneigte Position zu bringen, daß die Steine unter der Einwirkung der Schwerkraft eine stabile Lage einnehmen, die sie auch unter der Einwirkung der Stoßwellen beibehalten. Dies kann an sich durch ein Schwenken der Patientenliege um die weitere horizontale Achse erreicht werden, indem der Patient in eine Fuß- bzw. Beckenhochlage gebracht wird. Allerdings treten dabei solche Verlagerungen der Patientenliege relativ zu der Stoßwellenquelle und der Röntgen-Ortungseinrichtung auf, daß sich die Steine nicht mehr im Fokusbereich der Stoßwellen befinden. Es ist also erforderlich, die Steine vor der Weiterführung der Behandlung erneut zu orten und den Patienten derart auszurichten, daß sich die Steine wieder im Fokusbereich der Stoßwellen befinden.

Demgegenüber ist bei einer besonders vorteilhaften Ausführungsform der Erfindung, die die zusätzliche Aufgabe löst, Verlagerungen des therapeutisch relevanten Bereiches bzw. Zielgebietes relativ zu der Ortungseinrichtung beim Schwenken der Patientenliege zu vermeiden, vorgesehen, daß die als weitere Gerätekomponente vorgesehene und relativ zu den anderen Gerätekomponenten verstellbare Ortungseinrichtung wahlweise unabhängig von den anderen Gerätekomponenten oder synchron mit wenigstens einer der anderen Gerätekomponenten um die gemeinsame Schwenk achse schwenkbar ist. Die unabhängige Schwenkbarkeit der Ortungseinrichtung um die gemeinsame Schwenkachse kann nutzbar gemacht werden, um die Gerätekomponenten relativ zueinander so auszurichten, wie der jeweilige Behandlungsfall dies erfordert. Dabei ist sicherzustellen, daß der Fokusbereich der an den Körper des Lebewesens akustisch angekoppelten Stoßwellenquelle in einem mittels der Ortungseinrichtung georteten therapeutisch relevanten Bereich im Körper des Patienten liegt. Im Anschluß hierzu besteht infolge des Umstandes, daß die Ortungseinrichtung mit wenigstens einer der anderen Gerätekomponenten um die gemeinsame Schwenkachse synchron schwenkbar ist, die Möglichkeit, während der Behandlung die Patientenliege mit dem darauf liegenden Patienten entweder gemeinsam mit der Stoßwellenquelle oder gemeinsam mit der Ortungseinrichtung oder aber gemeinsam mit Ortungseinrichtung und Stoßwellenquelle in der jeweils erforderlichen Weise um die gemeinsame Schwenkachse zu schwenken, ohne daß sich der Fokusbereich der Stoßwellen aus dem einmal georteten therapeutisch relevanten Bereich verlagert und/oder sich der zunächst geortete therapeutisch relevante Bereich relativ zu der Ortungseinrichtung verlagert. Eine erneute Ortung des therapeutisch relevanten Bereiches nach dem Schwenkvorgang ist also in der Regel ebenso wie ein erneutes Ausrichten des Patienten vermieden. Zweckmäßigerweise verläuft die gemeinsame Schwenkachse im wesentlichen horizontal und/oder etwa quer zur Längsachse der Patientenliege.

Gemäß einer bevorzugten Variante der Erfindung ist vorgesehen, daß die Patientenliege und wenigstens eine andere Gerätekomponente ausgehend von einer waagerechten Lage der Patientenliege unabhängig von der Stellung der wenigstens einen anderen Gerätekomponente relativ zu der Patientenliege in wenigstens einer Richtung um zumindest 45° synchron schwenkbar sind. Hierdurch ist gewährleistet, daß unabhängig von der Winkellage, die die Stoßwellenquelle und/oder gegebenenfalls die Ortungseinrichtung bezüglich der gemeinsamen Schwenkachse in bezug auf die Patientenliege einnehmen, die Patientenliege mit wenigstens einer der anderen Gerätekomponente um ein solches Maß synchron um die gemeinsame Schwenkachse schwenkbar sind, daß eine gegenüber der horizontalen Lage der Patientenliege erheblich veränderte Schwerkraftwirkung auf im therapeutisch relevanten Bereich liegende Organe und/oder Konkremente auftritt. Es besteht im Falle des Vorhandenseins einer Ortungseinrichtung aber auch die Möglichkeit, zunächst durch synchrones Schwenken der Patientenliege mit der Ortungseinrichtung festzustellen, welche Auswirkungen auf die Lage eines im therapeutisch relevanten Bereich liegenden Organes und/oder Konkrementes infolge der Schwerkraftwirkung auftritt. Anschließend kann die Stoßwellenquelle, falls dies erforderlich ist, unabhängig von den beiden anderen Gerätekomponenten ebenfalls um die gemeinsame Achse geschwenkt werden. Außerdem besteht die Möglichkeit, die Patientenliege synchron mit der Stoßwellenquelle um die gemeinsame Achse zu schwenken und dabei mittels der Ortungseinrichtung, die ihre Ausrichtung im Raum beibehält, die durch das Schwenken hervorgerufenen Auswirkungen zu beobachten und eine Schwenkung der Ortungseinrichtung um die gemeinsame Achse nur vorzunehmen, falls dies erforderlich ist. Es besteht auch die Möglichkeit, Stoßwellenquelle und Ortungseinrichtung gemeinsam mit der Patientenliege um den genannten Winkel zu schwenken.

Eine weitere bevorzugte Variante der Erfindung sieht vor, daß unabhängig von der Stellung der Patientenliege, die diese bezüglich der gemeinsamen Schwenkachse einnimmt, zumindest eine der beiden anderen Gerätekomponenten um wenigstens 180° relativ zu der Patientenliege schwenkbar ist. Hierdurch wird erreicht, daß die Stoßwellenquelle und/oder gegebenenfalls die Ortungseinrichtung unabhängig von dem Neigungswinkel, den die Patientenliege relativ zu der Horizontalen einnimmt, wahlweise in eine Ober- oder Untertischposition gebracht werden können. Dabei wirken die Stoßwellen im Falle der Untertischposition der Stoßwellenquelle durch eine entsprechend geformte Öffnung der Patientenliege auf den Körper des Patienten ein. Das gleiche gilt, falls eine Ultraschall-Ortungseinrichtung in Untertischposition betrieben wird. Falls eine Röntgen-Ortungseinrichtung vorgesehen ist, besteht die Möglichkeit, den Röntgenstrahler wahlweise in Ober- oder Untertischposition zu bringen.

Besonders vorteilhafte Ausführungsformen der Erfindung sehen vor, daß die Ortungseinrichtung unabhängig von den beiden anderen Gerätekomponenten um ein Isozentrum sphärisch verstellbar ist und daß der mittels der Ortungseinrichtung ortbare therapeutisch relevante Bereich das Isozentrum enthält bzw. daß die Stoßwellenquelle unabhängig von der Patientenliege und gegebenenfalls der Ortungseinrichtung um ein Isozentrum sphärisch verstellbar ist und daß der Fokusbereich der Stoßwellen das Isozentrum enthält. Durch diese Maßnahme(n) können, nachdem die Patientenliege mit einem darauf liegenden Patienten einerseits und die Ortungseineichtung bzw. die Stoßwellenquelle andererseits relativ zueinander einmal so ausgerichtet sind, daß sich der therapeutisch relevante Bereich im Isozentrum befindet, die relative Ausrichtung der Ortungseinrichtung bzw. der Stoßwellenquelle relativ zu dem im Isozentrum liegenden therapeutisch relevanten Bereich verändert werden, ohne daß Verlagerungen des therapeutisch relevanten Bereiches relativ zu der Ortungseinrichtung bzw. Verlagerungen des Fokusbereiches aus dem therapeutisch relevanten Bereich auftreten. Falls sowohl die Ortungseinrichtung als auch die Stoßwellenquelle um ein Isozentrum sphärisch verstellbar sind, ist es zweckmäßig vorzusehen, daß die Stoßwellenquelle und die Ortungseinrichtung um das gleiche Isozentrum schwenkbar sind. Besonders vorteilhaft ist es außerdem, wenn die gemeinsame Schwenkachse gemäß einer Variante der Erfindung durch das Isozentrum verläuft, da dann einer der zur sphärischen bzw. isozentrischen Verstellung der Ortungseinrichtung bzw. der Stoßwellenquelle erforderlichen Freiheitsgrade durch die gemäß der Erfindung ohnehin erforderliche Schwenkbarkeit der Stoßwellenquelle um die gemeinsame Schwenkachse gegeben ist, was zu einer Reduzierung des konstruktiven Aufwandes führt. Der andere zur sphärischen bzw. isozentrischen Verstellbarkeit der Ortungseinrichtung bzw. der Stoßwellenquelle erforderliche Freiheitsgrad wird mit geringem konstruktiven Aufwand erreicht, wenn wenigstens für die Ortungseinrichtung bzw. die Stoßwellenquelle ein um die gemeinsame Schwenkachse schwenkbarer C-förmiger Träger vorgesehen ist, dessen Mittelachse parallel zu der gemeinsamen Schwenkachse verläuft und an dem die jeweilige Gerätekomponente derart angebracht ist, daß sie auf einer Kreisbahn um das jeweilige Isozentrum verstellbar ist. Dabei sieht eine Ausführung der Erfindung vor, daß die Mittelachse des die Stoßwellenquelle tragenden C-förmigen Trägers im Abstand von der gemeinsamen Schwenkachse verläuft und die Stoßwellenquelle derart an dem C-förmigen Träger angebracht ist, daß ihre akustische Achse parallel zur Mittelebene des C-förmigen Trägers verläuft und die gemeinsame Schwenkachse schneidet. Die Stoßwellenquelle ist also seitlich versetzt neben der Mittelebene des C-förmigen Trägers angeordnet, so daß die Zugänglichkeit zu dem Körper des Patienten im Bereich der Stoßwellenquelle nicht durch den C-förmigen Träger eingeschränkt ist. Eine weitere Ausführung sieht vor, daß die Radien der für die Ortungseinrichtung und die Stoßwellenquelle vorgesehenen C-förmigen Träger derart unterschiedlich gewählt sind, daß die Ortungseinrichtung und die Stoßwellenquelle kollisionsfrei übereinander hinweg um die gemeinsame Schwenkachse schwenkbar sind. Die kollisionsfreie Verstellbarkeit von Stoßwellenquelle und Ortungseinrichtung wird weiterhin begünstigt, wenn gemäß einer Ausführungsform der Erfindung die Mittelebenen der C-förmigen Träger derart unterschiedliche Abstände von der gemeinsamen Schwenkachse aufweisen, daß bei parallel zueinander ausgerichteten C-förmigen trägern ein Abstand zwischen den einander zugewandten Flächen der C-förmigen Träger vorliegt. Ausserdem ist durch diese Maßnahme gewährleistet, daß sich leicht eine geeignete Ausrichtung von Stoßwellenquelle und Ortungseinrichtung finden läßt, in der der die Stoßwellenquelle tragende C-förmige Träger die Funktion der Ortungseinrichtung nicht beeinträchtigt.

Gemäß einer Variante der Erfindung ist vorgesehen, daß der die Stoßwellenquelle tragende C-förmige Träger mit der Stoßwellenquelle in eine Parkstellung schwenkbar ist, in der die Mittelebene des C-förmigen Trägers im wesentlichen rechtwinklig zur gemeinsamen Schwenkachse verläuft, so daß der Patient auf die Patientenliege gebettet und von dieser abgenommen werden kann, ohne daß der C-förmige Träger eine Behinderung darstellt.

Außerdem wird das Umschwenken der Stoßwellenquelle von Ober- in Untertischlage und umgekehrt erleichtert, wenn die Stoßwellenquelle dazu mit dem C-förmigen Träger in ihre Parkposition gebracht wird.

Eine weitere Ausführungsform sieht vor, daß als Ortungseinrichtung ein einen Röntgenstrahler und einen diesem gegenüberliegenden Strahlenempfänger, z.B. einen Röntgenbildverstärker, aufweisendes Röntgendiagnostikgerät vorhanden ist, wobei mittels des Röntgenstrahlers ein Röntgenstrahlenbündel erzeugbar ist, dessen Zentralstrahl durch das Isozentrum zu dem Strahlenempfänger verläuft, und daß der die Röntgendiagnostikeinrichtung tragende C-förmige Träger als C-Bogen ausgeführt ist, an dessen Enden der Röntgenstrahler und der Strahlenempfänger angebracht sind, wobei der C-Bogen in einem Halter derart längs seines Umfanges verschiebbar aufgenommen ist, daß die Röntgendiagnostikeinrichtung um das Isozentrum schwenkbar ist, und wobei der C-Bogen um die gemeinsame Schwenkachse schwenkbar ist. Eine derartige Ortungseinrichtung dient dann, wenn bereits eine Ultraschall-Ortungseinrichtung, beispielsweise in Form eines in die Stoßwellenquelle integrierten Sektor-Scanners, vorhanden ist, zur Gewinnung zusätzlicher Informationen; sie kann auch ohne zusätzliche Ultraschall-Ortungseinrichtung betrieben werden, wenn der Patient zur Ortung des therapeutisch relevanten Bereiches mittels der Röntgendiangostikeinrichtung in an sich bekannter Weise unter unterschiedlichen Winkeln durchstrahlt wird. Gemäß einer Variante der Erfindung kann vorgesehen sein, daß die Mittelebene des C-Bogens die gemeinsame Schwenkachse enthält und daß die Mittelebene des die Stoßwellenquelle tragenden C-förmigen Trägers im Abstand von der gemeinsamen Schwenkachse verläuft und die Stoßwellenquelle derart an dem C-förmigen Träger angebracht ist, daß ihre akustische Achse parallel zu der Mittelebene des C-förmigen Trägers verläuft und den Zentralstrahl des Röntgenstrahlenbündels in der gemeinsamen Schwenkachse schneidet. Durch diese Maßnahme ist die Gefahr, daß sich die Stoßwellenquelle oder der diese tragende C-förmige Träger im Strahlengang der Ortungseinrichtung befindet, auf ein Mindestmaß verringert.

Es ist zweckmäßig, wenn die Patientenliege relativ zu der gemeinsamen Schwenkachse in Richtung der Achsen eines räumlichen rechtwinkligen Koordinatensystems verstellbar ist, wobei eine Achse des Koordinatensystems parallel zur Längsachse der Patientenliege und eine andere Achse des Koordinatensystems parallel zu der gemeinsamen Schwenkachse verläuft. Hierdurch ist es einfach möglich, den Patienten durch Verstellen der Patientenliege so auszurichten, daß sich der therapeutisch relevante Bereich im Isozentrum befindet bzw. der Fokusbereich der Stoßwellen im therapeutisch relevanten Bereich liegt. Die Patientenliege ist gemäß einer Variante der Erfindung an einem L-förmigen Träger angebracht, der mit seinem einen Ende um die gemeinsame Schwenkachse schwenkbar gelagert ist und an dessen anderem Ende die Patientenliege freitragend befestigt ist. Es ergibt sich so eine gute Zugänglichkeit zu der Patientenliege bzw. einem auf dieser liegenden Patienten.

Eine bevorzugte Verwendung des erfindungsgemäßen Gerätes ist die berührungslose Zertrümmerung von im Körper eines Lebewesens befindlichen Konkrementen.

Ausführungsbeispiele der Erfindung sind in den beigefügten Zeichnungen dargestellt. Es zeigen:

Fig. 1 eine perspektivische Ansicht eines erfindungsgemäßen Gerätes,

Fig. 2 eine schematische Ansicht des Gerätes in Richtung des Pfeiles A gemäß Fig. 1,

Fig. 3 eine schematische Ansicht des Gerätes in Richtung des Pfeiles B gemäß Fig. 1,

Fig. 4 in grob schematischer Darstellung ein Blockschaltbild des erfindungsgemäßen Gerätes,

Fig. 5 eine schematische einen von Fig. 1 abweichenden Betriebszustand verdeutlichende teilweise Ansicht des Gerätes in Richtung des Pfeiles C gemäß Fig. 1 ohne Patientenliege,

Fig. 6 den Betriebszustand gemäß Fig. 5 in einer schematischen teilweisen Ansicht des Gerätes in Richtung des Pfeiles D gemäß Fig. 1 ohne Patientenliege,

Fig. 7 eine schematische einen weiteren Betriebszustand des Gerätes verdeutlichende teilweise Ansicht in Richtung des Pfeiles D gemäß Fig. 1 ohne Patientenliege, und

Fig. 8 bis 11 in zu den Fig. 1 bis 4 analoger Darstellung eine weitere Ausführungsform eines erfindungsgemäßen Gerätes.

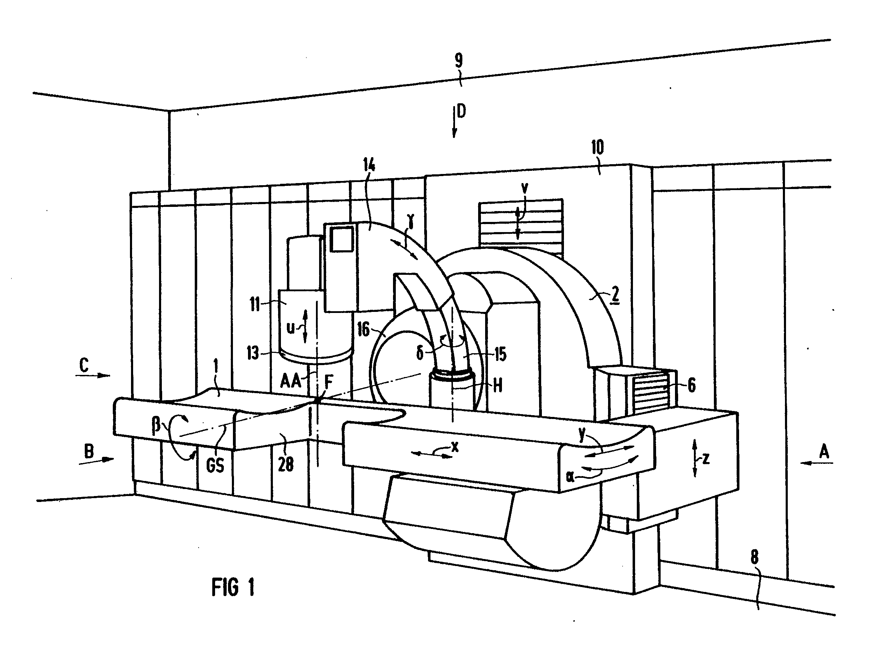

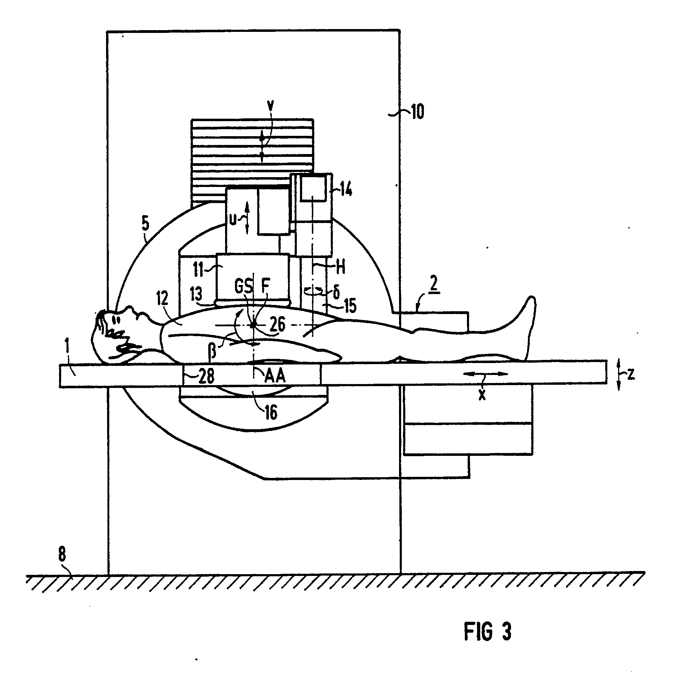

Wie aus den Fig. 1 bis 3 deutlich wird, besitzt das Gerät eine Patientenliege 1, die im Bereich ihres einen Endes, es handelt sich hierbei um das Fußende, freitragend an einem L-förmigen Träger 2 angebracht ist. Der L-förmige Träger 2 besitzt einen ersten Schenkel 3, an dessen freiem Ende die Patientenliege 1 befestigt ist. Der erste Schenkel 3 weist zwei etwa kastenförmige Schenkelabschnitte 4a, 4b rechteckigen Querschnittes auf, die unter Zwischenschaltung einer nicht dargestellten geeigneten Führung teleskopartig derart ineinandergefügt sind, daß die an dem Schenkelabschnitt 4a befestigte Patientenliege 1 quer zur Richtung ihrer Längsachse in Richtung des Doppelpfeiles y geradlinig verstellbar ist. Die Patientenliege 1 ist an dem freien Ende des L-förmigen Trägers bzw. des Schenkelabschnittes 4a mittels geeigneter nicht dargestellter Führungsmittel derart angebracht, daß sie in Richtung ihrer Längsachse, also in Richtung des Doppelpfeiles y geradlinig verstellbar ist. Außerdem ist die Patientenliege 1 an dem freien Ende des ersten Schenkels 3 bzw. des Schenkelabschnittes 4a mittels geeigneter ebenfalls nicht dargestellter Gelenkmittel derart angebracht, daß sie um eine oberhalb der Patientenliege und parallel zu deren Längsachse verlaufende Achse in Richtung des gekrümmten Doppelpfeiles "Alpha" kippbar ist.

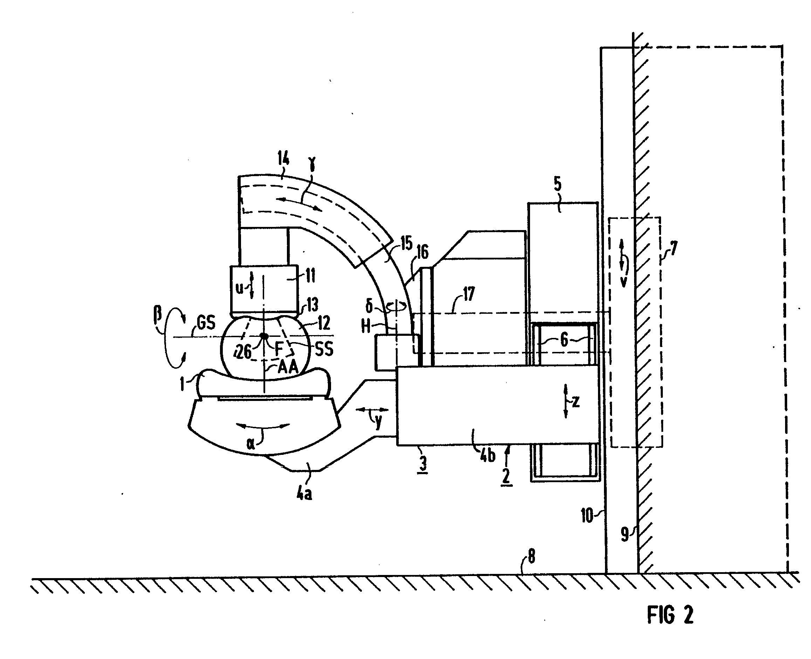

Der erste Schenkel 3 des L-förmigen Trägers 2 ist mit seinem Schenkelabschnitt 4b mittels einer geeigneten, in Fig. 2 angedeuteten Führung 6 mit dem etwa kreisscheibenförmig ausgebildeten zweiten Schenkel 5 des L-förmigen Trägers 2 in Richtung des Doppelpfeiles z geradlinig verstellbar verbunden, so daß die Patientenliege in einer rechtwinklig zu ihrer Ebene verlaufenden Richtung verstellbar ist. Der zweite Schenkel 5 des L-förmigen Trägers 2 ist an einem in Fig. 2 angedeuteten Wagen 7 angebracht, der seinerseits mittels einer geeigneten nicht dargestellten Führung an einem auf dem Boden 8 des Untersuchungsraumes aufstehenden und in dessen Wand eingelassenen Ständer 10 in Richtung des Doppelpfeiles v, also in vertikaler Richtung, geradlinig verstellbar angebracht ist.

Das Gerät weist außerdem eine schematisch angedeutete Stoßwellenquelle 11 auf, mittels derer Stoßwellen erzeugbar sind, die in einem auf der akustischen Achse AA der Stoßwellenquelle 11 liegenden Fokusbereich F zusammenlaufen. Bei der Stoßwellenquelle 11 handelt es sich beispielsweise um eine elektro-dynamische Stoßwellenquelle, wie sie in der DE-OS 33 28 051 beschrieben ist. Die Stoßwellenquelle 11 enthält eine in den Fig. 1 bis 3 nicht dargestellte Ultraschall-Ortungseinrichtung, die wenigstens einen Ultraschall-Sektor-Scanner aufweist, mittels dessen eine die akustische Achse AA und den Fokusbereich F enthaltende in Fig. 2 angedeutete kreissektorförmige Schicht SS des Körpers des Patienten 12 abtastbar ist. Eine Stoßwellenquelle mit integrierter Ultraschall-Ortungseinrichtung ist in der DE-OS 37 25 533 beschrieben. Die Stoßwellenquelle 11 besitzt einen mittels eines flexiblen Balges 13 abgeschlossenen, mit einem akustischen Ausbreitungsmedium für die Stoßwellen, z.B. Wasser, gefüllten Raum und wird an den Körper des Patienten 12 akustisch angekoppelt, indem sie mit dem Balg 13 gegen die Körperoberfläche des Patienten gepreßt wird, so wie dies in Fig. 2 dargestellt ist.

Die Stoßwellenquelle 11 ist an einem Schlitten 14 angebracht, der mittels einer geeigneten nicht dargestellten Führung auf einem kreisbogenförmig gekrümmten, C-förmigen Träger 15 etwa quadratischen Querschnittes geführt ist. Der die Stoßwellenquelle 11 tragende Schlitten 14 ist längs des Umfanges des sich über etwa 90° erstreckenden C-förmigen Trägers 15 in Richtung des gekrümmten Doppelpfeiles "Gamma" verstellbar. Dabei liegt im normalen Betrieb des Gerätes der Fokusbereich F der Stoßwellen auf der Mittelachse des C-förmigen Trägers 15. Die Stoßwellenquelle 11, deren akustische Achse AA die Mittelachse des C-förmigen Trägers 15 im rechten Winkel schneidet, ist also in einer solchen Weise auf einer Kreisbahn um die Mittelachse des C-förmigen Trägers 15 schwenkbar, daß der Fokusbereich F der Stoßwellen stets auf der Mittelachse des C-förmigen Trägers 15 und die akustische Achse AA in einer die Mittelachse des C-förmigen Trägers 15 enthaltenden Ebene liegt. Die Stoßwellenquelle 11 ist derart an dem Schlitten 14 angebracht, daß ihre akustische Achse AA seitlich versetzt im Abstand zu der Mittelebene des C-förmigen Trägers 15 und parallel zu dieser verläuft. Dabei ist die Stoßwellenquelle 11 mittels einer nicht dargestellten Führung in Richtung des Doppelpfeiles u geradlinig parallel zu ihrer akustischen Achse AA verschiebbar angebracht, wobei in der radial innersten Position der Stoßwellenquelle 11 der Fokusbereich F der Stoßwellen auf der Mittelachse des C-förmigen Trägers 15 liegt.

Durch die seitlich versetzte Montage der Stoßwellenquelle 11 an dem Schlitten 14 wird erreicht, daß die Zugänglichkeit zu demjenigen Bereich des Körpers des Patienten 12, in dem die Stoßwellenquelle 11 an diesen angekoppelt ist, nicht durch den C-förmigen Träger 15 eingeschränkt wird. Ein Umbetten eines auf der Patientenliege 1 liegenden Patienten 12 wird erleichtert, wenn die Stoßwellenquelle 11 in Richtung des Doppelpfeiles u in ihre radial äußerst Position verfahren wird.

Der C-förmige Träger 15 ist mit einem Tragteil 16 verbunden, das ebenso wie der zweite Schenkel 5 des L-förmigen Trägers 2 an dem in Richtung des Doppelpfeiles v geradlinig verstellbar an dem Ständer 10 angebrachten Wagen 7 angebracht ist. Die Patientenliege 1 und die Stoßwellenquelle 11 sind also gemeinsam durch Verfahren des Wagens 7 an dem Ständer 10 in Richtung des Doppelpfeiles v verstellbar.

Sowohl die Stoßwellenquelle 11 als auch die Patientenliege 1 sind zusätzlich zu den bereits beschriebenen Verstellmöglichkeiten um eine horizontal und quer zur Längsachse der Patientenliege 1 verlaufende gemeinsame Schwenkachse GS schwenkbar, die im normalen Betrieb des Gerätes, d.h., wenn die Stoßwellenquelle 11 ihre radial innerste Position einnimmt, durch den Fokusbereich F der Stoßwellen verläuft. Dies wird dadurch erreicht, daß an dem Wagen 7 eine in Fig. 2 strichliert angedeutete Achse 17 angebracht ist, auf der zum einen der zweite Schenkel 5 des die Patientenliege 1 tragenden L-förmigen Trägers 2 und zum anderen das den C-förmigen Träger 15 mit der Stoßwellenquelle 11 tragende Tragteil 16 in nicht näher dargestellter Weise in Richtung der gekrümmten Doppelpfeiles "Beta" schwenkbar gelagert sind. Dabei ist der C-förmige Träger 15 in bezug auf die gemeinsame Schwenkachse GS um das erforderliche Maß exzentrisch versetzt an dem Tragteil 16 angebracht, um zu erreichen, daß die wie beschrieben im Abstand von der Mittelebene des C-förmigen Trägers 15 und parallel zu dieser verlaufende akustische Achse AA der Stoßwellenquelle 11 die gemeinsame Schwenkachse GS schneidet. Der C-förmige Träger 15 nimmt im normalen Betrieb des Gerätes eine solche Lage ein, daß seine Mittelebene parallel zu der gemeinsamen Schwenkachse GS verläuft.

Infolge der beschriebenen Schwenkbarkeit des C-förmigen Trägers 15 mit der Stoßwellenquelle 11 um die gemeinsame Schwenkachse sowie infolge der beschriebenen Verstellbarkeit der Stoßwellenquelle 11 längs des Umfanges des C-förmigen Trägers 15 auf einer Kreisbahn ist erreicht, daß die Stoßwellenquelle 11 um den ein auf der gemeinsamen Schwenkachse GS liegendes Isozentrum bildenden Fokusbereich F sphärisch verstellbar ist. Im folgenden wird daher von einer isozentrischen Verstellbarkeit der Stoßwellenquelle 11 gesprochen.

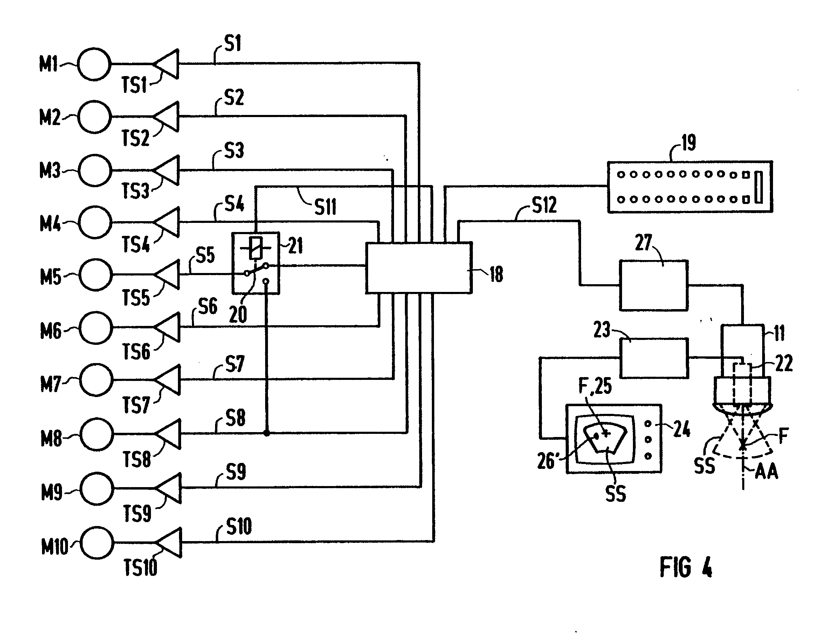

Um die beschriebenen Verstellbewegungen ausführen zu können, sind in den Fig. 1 bis 3 nicht dargestellte Motore M1 bis M10 vorhanden, die unter Zwischenschaltung ebenfalls nicht dargestellter geeigneter Getriebe auf das jeweils zu verstellende Teil einwirken. Die Motore M1 bis M10 sind in Fig. 4 schematisch angedeutet. Dabei dient der Motor M1 zur Verstellung der Patientenliege in Richtung des Doppelpfeiles x, der Motor M2 zur Verstellung des Schenkelabschnittes 4a des ersten Schenkels 3 mit der Patientenliege 1 in Richtung des Doppelpfeiles y, der Motor M3 zur Verstellung des ersten Schenkels 3 mit der Patientenliege 1 in Richtung des Doppelpfeiles 2, der Motor M4 zur Kippung der Patientenliege 1 in Richtung des gekrümmten Doppelpfeiles "Alpha" und der Motor M5 zum Schwenken des L-förmigen Trägers 2 mit der Patientenliege 1 um die gemeinsame Schwenkachse GS in Richtung des gekrümmten Doppelpfeiles "Beta".

Der Motor M6 bewirkt die Verstellung des Wagens 7 und damit der Patientenliege 1 und der Stoßwellenquelle 11 in Richtung des Doppelpfeiles v.

Der Motor M7 dient zur Verstellung des Schlittens 14 mit der Stoßwellenquelle 11 in Richtung des gekrümmten Doppelpfeiles "Gamma", der Motor M8 bewirkt die Verstellung der Stoßwellenquelle 11 in Richtung des Doppelpfeiles u und der Motor M9 dient zum Schwenken des Tragteiles 16 mit dem C-förmigen Träger 15 und der Stoßwellenquelle 11 um die gemeinsame Schwenkachse GS in Richtung des gekrümmten Doppelpfeiles "Beta".

Der Zweck des in Fig. 4 außerdem dargestellten Motors M10 wird im folgenden noch erläutert.

Die Motore M1 bis M10 sind unter Zwischenschaltung von schematisch angedeuteten Treiberschaltungen TS1 bis TS10 über Steuerleitungen S1 bis S10 mit einer Steuereinheit 18 verbunden, an die eine Bedieneinheit 19 angeschlossen ist. Die Bedieneinheit 19 besitzt für jeden der Motore M1 bis M10 zwei Tasten, von denen die eine dazu dient, den jeweiligen Motor, solange sie gedrückt ist, in Vorwärtsrichtung laufen zu lassen, und die andere dazu dient, den jeweiligen Motor, solange sie gedrückt ist, in Rückwärtsrichtung laufen zu lassen. Die Motore M5 und M8 und die entsprechenden Getriebe sind so gewählt, daß die Verstellung der Patientenliege 1 bzw. der Stoßwellenquelle 11 in Richtung des gekrümmten Doppelpfeiles "Beta" jeweils exakt mit der gleichen Winkelgeschwindigkeit erfolgt. In der von der Steuereinheit 18 zu der zu dem Motor M5 gehörigen Treiberstufe TS5 führenden Steuerleitung S5 liegt der Umschaltkontakt 20 eines Relais 21, dessen Erregerspule über eine Steuerleitung S11 mit der Steuereinheit 18 verbunden ist. Im Normalfall nimmt der Umschaltkontakt 20 die in Fig. 4 gezeigte Schaltstellung ein. Wird jedoch eine von zwei zusätzlichen Tasten der Bedieneinheit 19, die sich von den den Motoren M1 bis M10 zugeordneten Tasten durch ihre andersartige Form unterscheiden, betätigt, wird das Relais 21 über die zugehörige Steuerleitung S11 von der Steuereinheit 18 derart angesteuert, daß sich der Schaltzustand des Umschaltkontaktes 20 ändert. Die zu dem Motor M5 gehörige Treiberstufe TS5 ist dann mit der zu dem Motor M8 gehörenden Treiberstufe TS 8 führenden Steuerleitung S8 verbunden, welche in Abhängigkeit davon, welche der beiden zusätzlichen Tasten gedrückt ist, ein Steuersignal führt, das eine Drehung der Motore M5 und M8 in der einen oder der anderen Richtung bewirkt. Solange eine der beiden zusätzlichen Tasten gedrückt ist, werden also die zum Schwenken der Patientenliege 1 bzw. der Stoßwellenquelle 11 dienenden Motore M5 und M8 derart gemeinsam aktiviert, daß die Patientenliege 1 und die Stoßwellenquelle 11 in der einen oder der anderen Richtung in Richtung des gekrümmten Doppelpfeiles "Beta" synchron um die gemeinsame Schwenkachse GS geschwenkt werden. Es wird also deutlich, daß die Patientenliege 1 und die Stoßwellenquelle 11 wahlweise unabhängig voneinander oder synchron um die gemeinsame Schwenkachse GS schwenkbar sind. Dabei ist es aus Gründen, die noch erläutert werden, zweckmäßig, wenn die Patientenliege 1 und die Stoßwellenquelle 11 - unabhängig von der Stellung, die die Stoßwellenquelle 11 relativ zu der Patientenliege 1 einnimmt - ausgehend von der in den Fig. 1 bis 3 dargestellten, bezüglich der gemeinsamen Schwenkachse GS horizontalen Lage der Patientenliege 1 wenigstens in einer Richtung um zumindest 45° synchron schwenkbar sind. Im Falle des beschriebenen Ausführungsbeispieles ist in beiden Richtungen eine synchrone Schwenkbarkeit von 90° vorgesehen.

In Fig. 4 ist außerdem die Stoßwellenquelle 11 mit einem in diese integrierten Ultraschall-Sektor-Scanner 22 angedeutet. Der Ultraschall-Sektor-Scanner 22 ist unter Zwischenschaltung einer an sich bekannten Bilderzeugungs- und Steuerelektronik 23 mit einem Monitor 24 verbunden, der zur Darstellung der mittels des Ultraschall-Sektor-Scanners 22 abgetasteten, den Fokusbereich F enthaltenden Schicht SS des Körpers des Patienten 12 dient. Die Lage des Fokusbereiches F ist durch eine mittels der Bilderzeugungs- und Steuerelektronik 23 in das Monitorbild eingeblendeten Marke 25 gekennzeichnet. In Fig. 4 ist außerdem das Bild 26′ eines in Fig. 2 bei 26 angedeuteten zu zertrümmernden Konkrementes im Monitorbild angedeutet.

Außerdem ist in Fig. 4 eine mit der Stoßwellenquelle 11 verbundene Generatorschaltung 27 angedeutet, die dazu dient, die Stoßwellenquelle 11 zur Erzeugung von Stoßwellen anzutreiben. Die Generatorschaltung 27 ist über eine Steuerleitung S12 mit der Steuereinheit 18 verbunden. Wird eine sich durch ihre Form und Größe von den übrigen Tasten unterscheidende Taste der Bedieneinheit 19 betätigt, veranlaßt die Steuereinheit 18 die Generatorschaltung 27, die Stoßwellenquelle 11 zur Abgabe einer vorwählbaren Anzahl von aufeinanderfolgenden Stoßwellen anzutreiben.

Um ein Konkrement 26, z.B. einen Nieren- oder einen Gallenblasenstein, im Körper eines Patienten 12 zu zertrümmern, wird beispielsweise so vorgegangen, daß zunächst die Patientenliege 1 in eine horizontale Position und durch Verstellen des Wagens 7 in ihre tiefste Position gebracht wird. Anschließend wird die Stoßwellenquelle 11 in Richtung des Doppelpfeiles u in ihre radial äußerste Position gefahren. Der Patient 12 wird dann auf die Patientenliege 1 gebettet, und zwar so, daß sich das Zielgebiet, also das das Konkrement 26 aufweisende Organ, etwa unterhalb der Stoßwellenquelle 11 befindet. Dabei kann es zweckmäßig sein, die Patientenliege 1 um ein gewisses Maß in Richtung des gekrümmten Doppelpfeiles "Alpha" in die eine oder andere Richtung zu kippen, um zu erreichen, daß das zu zertrümmernde Konkrement 26 infolge der Schwerkraftwirkung in dem jeweiligen Organ eine stabile Lage einnimmt. Im Anschluß daran wird die Patientenliege 1 durch Verstellen des Wagens 7 in eine dem behandelnden Arzt angenehme Höhe gebracht. Außerdem wird die Stoßwellenquelle 11 in Richtung des Doppelpfeiles u in ihre radial innerste Position verstellt, in der der Fokusbereich F auf der gemeinsamen Schwenkachse GS liegt. Dabei wird der flexible Balg 13 der Stoßwellenquelle 11 an die Körperoberfläche des Patienten 12, die zuvor zur Verbesserung der akustischen Ankoppelung mit einem Ultraschallgel oder dergleichen bestrichen wurde, angepreßt. Nun wird die Patientenliege 1 in Richtung des Doppelpfeiles x so lange hin und her verstellt, bis das zu zertrümmernde Konkrement 26 in der mittels des Ultraschall-Sektor-Scanners 22 abgetasteten Schicht SS liegt und demzufolge das Bild 26′ des Konkrementes 26 auf dem Monitor 24 sichtbar ist. Vorzugsweise ist der Ultraschall-Sektor-Scanner 22 dabei derart ausgerichtet, daß die abgetastete Schicht SS die gemeinsame Schwenkachse GS enthält. Falls der Patient 12 zu Beginn in einer ungünstigen Lage auf die Patientenliege 1 gebettet wurde, kann es erforderlich sein, die Patientenliege 1 zusätzlich in Richtung der Doppelpfeile z und/oder y zu verstellen, um zu erreichen, daß das zu zertrümmernde Konkrement 26 in die mittels des Ultraschall-Sektor-Scanners 22 abtastbare Schicht SS gebracht werden kann. Erscheint das Bild 26′ des zu zertrümmernden Konkrementes 26 auf dem Monitor 24, wird der Patient 12 durch Verstellen der Patientenliege 1 in Richtung der Doppelpfeile y und/oder z derart positioniert, daß sich das Bild 26′ des zu zertrümmernden Konkrementes 26 mit der der Position des Fokusbereiches F entsprechenden Marke 25 in dem Bild des Monitors 24 deckt. Ausgehend von dieser Position be steht nun die Möglichkeit, die Stoßwellenquelle 11 durch Verstellen des Schlittens 14 in Richtung des Doppelpfeiles "Gamma" und/oder durch Schwenken des C-förmigen Trägers 15 um die gemeinsame Schwenkachse GS in Richtung des gekrümmten Doppelpfeiles "Beta" relativ zu dem Patienten 12 so auszurichten, daß sich im Ausbreitungsweg der Stoßwellen weder Hindernisse, wie etwa Knochen, noch Organe, wie z.B. die Lunge, befinden, die durch die Wirkung der Stoßwellen geschädigt werden könnten. Dabei besteht infolge des Umstandes, daß die Stoßwellenquelle 11 isozentrisch um den Fokusbereich F verstellt wird, keinerlei Gefahr, daß sich der Fokusbereich F in einer solchen Weise verlagert, daß er außerhalb des zu zertrümmernden Konkrementes 26 zu liegen kommt. Ist die optimale Ausrichtung der Stoßwellenquelle 11 gefunden, kann mit der Stoßwellenbehandlung begonnen werden. Falls dies erforderlich ist, kann vor oder während der Behandlung ein synchrones Schwenken der Patientenliege 1 und der Stoßwellenquelle 11 um die gemeinsame Schwenkachse GS in Richtung des gekrümmten Doppelpfeiles "Beta" erfolgen, um die Lage eines zu zertrümmernden Konkrementes durch Schwerkraftwirkung zu stabilisieren. Auch dabei treten keinerlei Verlagerungen des Fokus F auf. Es kann allenfalls erforderlich sein, die Ausrichtung des Patienten 12 relativ zu der Stoßwellenquelle 11 durch Verstellen der Patientenliege 1 geringfügig zu korrigieren, um einer eventuellen Veränderung der Lage des Konkrementes 26 Rechnung zu tragen. Vor dem Schwenken der Patientenliege 1 um die gemeinsame Schwenkachse GS ist es, insbesondere bei stark von der Horizontalen abweichenden Schwenkstellungen - der Patient 12 kann infolge der synchronen Schwenkbarkeit der Patientenliege 1 mit der Stoßwellenquelle 11 um ± 90° um die gemeinsame Schwenkachse GS beispielsweise in eine stehende Position gebracht werden - zweckmäßig, eine Fußbank an der Patientenliege 1 anzubringen und den Patienten 12 durch Gurte oder dergleichen zu fixieren.

Nach Abschluß der Behandlung wird die Patientenliege erforderlichenfalls wieder abgesenkt, in ihre horizontale Ausgangslage gebracht und die Stoßwellenquelle 11 in ihre radial äußerste Position verstellt, um den Patienten bequem von der Patientenliege 1 abnehmen zu können.

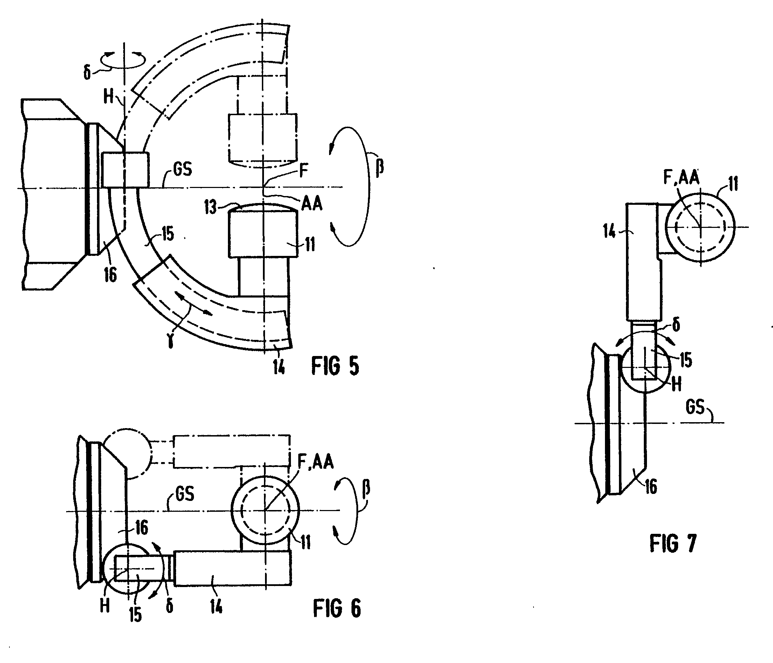

Für bestimmte Behandlungsfälle, vorzugsweise bei der Zertrümmerung von Nierenstienen eines in Rückenlage auf der Patientenliege 1 liegenden Patienten 12, ist es erforderlich, die in den Fig. 1 bis 3 in ihrer Obertischposition dargestellte Stoßwellenquelle 11 in eine Untertischposition bringen zu können, in der sie durch einen in Fig. 1 dargestellten Ausschnitt 28, der beim Betrieb des Gerätes mit in ihrer Obertischposition befindlicher Stoßwellenquelle 11 in nicht dargestellter Weise mittels eines geeignet geformten Verschlußstückes verschließbar ist, an den Körper eines auf der Patientenliege 1 liegenden Patienten akustisch angekoppelt wird. Zu diesem Zweck kann der die Stoßwellenquelle 11 tragende C-förmige Träger 15 unabhängig von der jeweiligen Schwenkposition, die die Patientenliege bezüglich der gemeinsamen Schwenkachse GS in Richtung des gekrümmten Doppelpfeiles "Beta" einnimmt, um wenigstens 180° in die in den Fig. 5 und 6 dargestellte Position geschwenkt werden, wobei die Position der Stoßwellenquelle 11 gemäß den Fig. 1 bis 3 strichliert angedeutet ist. Um dies bewerkstelligen zu können, ist der C-förmige Träger 15 mittels des Motors M10 um eine in eine zu der gemeinsamen Schwenkachse GS senkrecht verlaufenden Ebene angeordnete Achse H in Richtung des gekrümmten Doppelpfeiles "Delta" schwenkbar an dem Tragteil 16 angebracht, und zwar derart, daß der C-förmige Träger 15 mit der Stoßwellenquelle 11, so wie dies in Fig. 7 dargestellt ist, in eine Parkposition schwenkbar ist, in der die Mittelebene des C-förmigen Trägers 15 etwa rechtwinklig zur gemeinsamen Schwenkachse GS verläuft. Wird die Patientenliege 1 nun in Richtung des Doppelpfeiles y in ihre von dem Ständer 10 entfernteste Position gebracht, kann die in ihrer Parkposition befindliche Stoßwellenquelle 11 kollisionsfrei an der Patientenliege 1 von ihrer Obertisch- in ihre Untertischposition und umgekehrt geschwenkt werden. Um Bodenberührungen der Stoßwellenquelle 11 bzw. des C-förmigen Trägers 15 zu vermeiden, wird dabei die Patientenliege 1 durch Verstellen des Wagens 7 in eine solche Höhe gebracht, daß eine ausreichende Bodenfreiheit vorliegt. Es versteht sich, daß die Stoßwellenquelle 11 für den Betrieb des Gerätes wieder aus ihrer Parkposition herausgeschwenkt werden muß. Übrigens kann die Stoßwellenquelle 11 auch in ihre Parkposition gebracht werden, um das Auflegen eines Patienten 12 auf die Patientenliege 1 bzw. das Abnehmen des Patienten 12 von der Patientenliege 1 zu erleichtern. Es versteht sich, daß dann, wenn die Stoßwellenquelle 11 ihre Untertischposition einnimmt, die Verstellbarkeit der Patientenliege 1 in Richtung der Doppelpfeile x und y durch die Abmessungen des Ausschnittes 28 begrenzt ist.

Im Falle des beschriebenen Ausführungsbeispieles ist die Stoßwellenquelle 11 um den Fokusbereich F als auf der gemeinsamen Schwenkachse GS liegendes Isozentrum sphärisch verstellbar. Das beschriebene synchrone Schwenken von Patientenliege 1 und Stoßwellenquelle 11 um die gemeinsame Schwenkachse GS ist ohne Verlagerungen des Fokusbereiches F jedoch auch dann möglich, wenn keine isozentrische, sondern eine andere Verstellbarkeit der Stoßwellenquelle 11 relativ zu der Patientenliege 1 gegeben ist, und wenn der Fokusbereich F nicht auf der gemeinsamen Schwenkachse GS liegt.

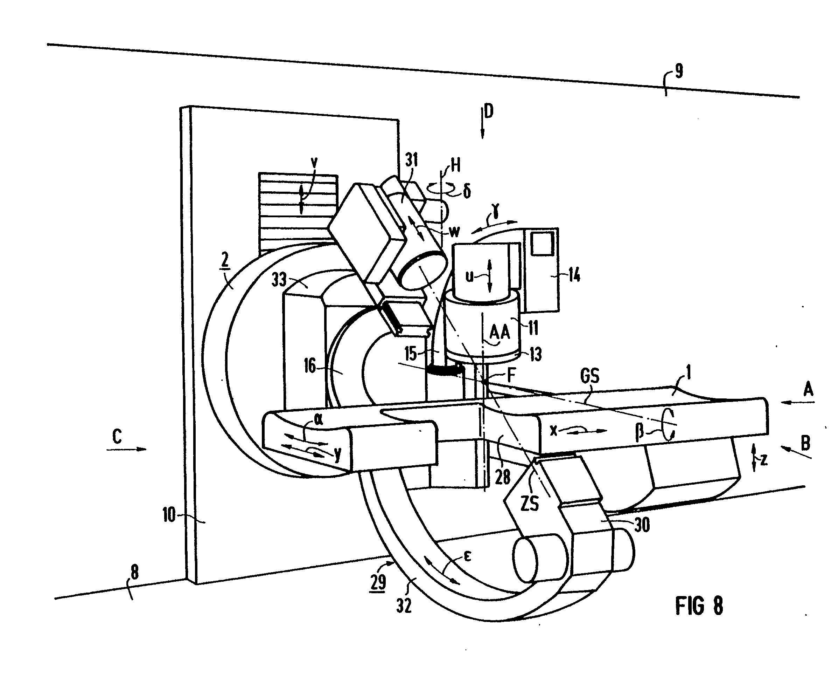

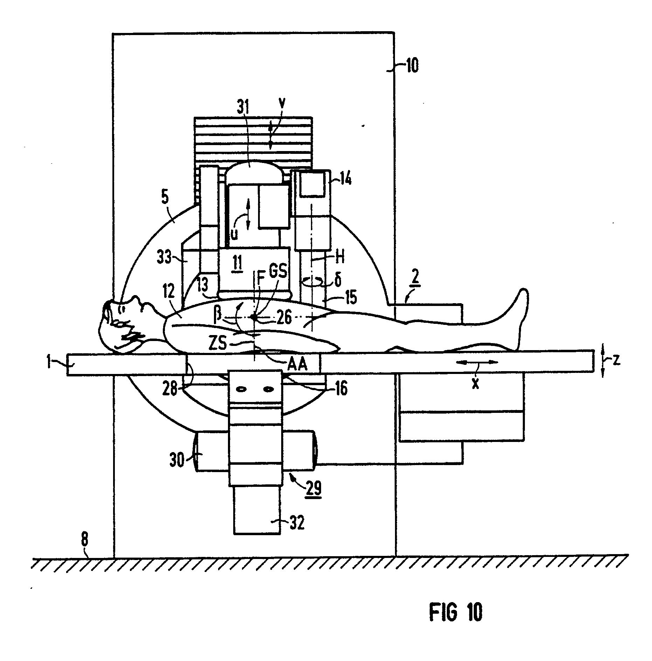

Das in den Fig. 8 bis 11 dargestellte erfindungsgemäße Gerät stimmt mit dem zuvor beschriebenen in wesentlichen Punkten überein, weshalb gleiche Teile die jeweils gleichen Bezugszeichen tragen. Der wesentliche Unterschied zu dem zuvor beschriebenen Ausführungsbeispiel besteht darin, daß das Gerät nach den Fig. 8 bis 11 außer dem Ultraschall-Sektor-Scanner 22 als weitere Ortungseinrichtung eine insgesamt mit 29 bezeichnete Röntgendiagnostikeinrichtung aufweist. Diese weist ihrerseits einen Röntgenstrahler 30, einen Röntgenbildverstärker 31 und einen C-Bogen 32 aufweist, der die im Bereich ihrer Auflagefläche für den Patienten röntgenstrahlendurchlässige Patientenliege 1 umgreift. Mittels des Röntgenstrahlers 30 ist ein Röntgenstrahlenbündel erzeugbar, dessen Zentralstrahl ZS mittig durch den Eingangsleuchtschirm des Röntgenbildverstärkers 31 verläuft. Der Röntgenstrahler 30 und der Röntgenbildverstärker 31 sind einander gegenüberliegend an den Enden des sich über etwa 180° erstreckenden C-Bogens 32 derart angebracht, daß der Zentralstrahl ZS des Röntgenstrahlenbündels in der Mittelebene des C-Bogens 32 verläuft und dessen Mittelachse im rechten Winkel schneidet. Der C-Bogen 32 ist mittels geeigneter nicht dargestellter Führungsmittel in einem Halter 33 derart verstellbar aufgenommen, daß er längs seines Umfanges um seine Mittelachse in Richtung des gekrümmten Doppelpfeiles "Epsilon" schwenkbar ist. Dabei werden der Röntgenstrahler 30 und der Bildverstärker 31 auf einer Kreisbahn um die Mittelachse des C-Bogens 32 geschwenkt, wobei der Zentralstrahl ZS des Röntgenstrahlenbündels stets die Mittelachse des C-Bogens 32 schneidet. Der Röntgenbildverstärker 31 ist mittels geeigneter nicht dargestellter Führungsmittel derart an dem C-Bogen 32 angebracht, daß er in Richtung des Doppelpfeiles w geradlinig parallel zum Zentralstrahl ZS des Röntgenstrahlenbündels verschiebbar ist. Es ist so möglich, den Röntgenbildverstärker 31 jeweils möglichst dicht bei der Körperoberfläche eines auf der Patientenliege 1 liegenden Patienten 12 zu positionieren.

Das mit dem C-förmigen Träger 15 verbundene Tragteil 16 und der den C-Bogen 32 aufnehmende Halter 33 sind ebenso wie der zweite Schenkel 5 des L-förmigen Trägers 2 an dem in Richtung des Doppelpfeiles v geradlinig an dem Ständer 10 verstellbaren Wagen 7 angebracht. Die Patientenliege 1, die Stoßwellenquelle 11 und die Röntgendiagnostikeinrichtung 29 sind also gemeinsam durch Verfahren des Wagens 7 an dem Ständer 10 in Richtung des Doppelpfeiles v verstellbar.

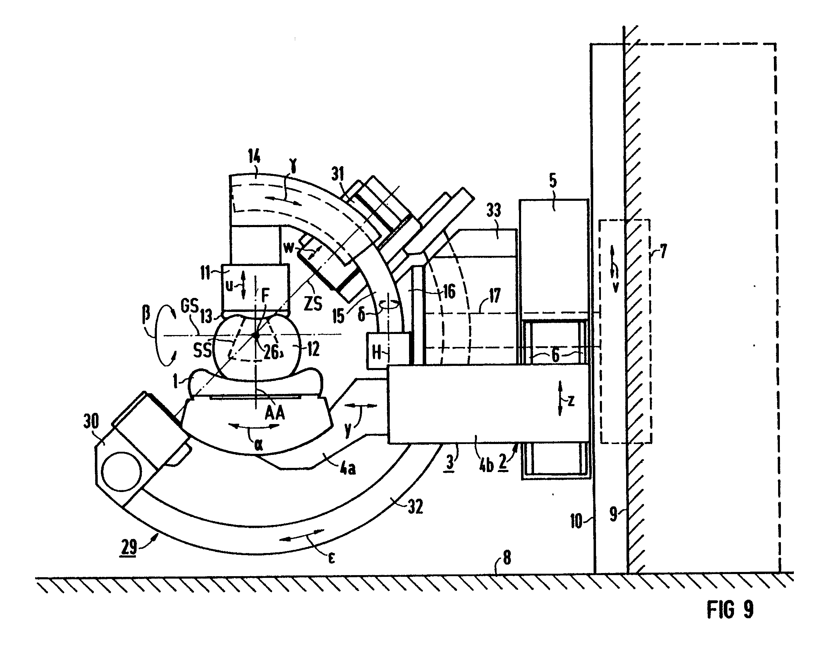

Sowohl für die Patientenliege 1 als auch für die Stoßwellenquelle 11 sind die bei dem zuvor beschriebenen Ausführungsbeispiel vorhandenen Verstellmöglichkeiten gegeben, das heißt, daß diese beiden Gerätekomponenten unter anderem um die gemeinsame Schwenkachse GS schwenkbar. Diese verläuft im normalen Betrieb des Gerätes, d.h., wenn die Stoßwellenquelle 11 ihre radial innerste Position einnimmt, wieder durch den Fokusbereich F der Stoßwellen und schneidet außerdem den Zentralstrahl ZS des Röntgenstrahlenbündels, der seinerseits durch den Fokusbereich F der Stoßwellen verläuft. Auch die Röntgendiagnostikeinrichtung 29 ist um die gemeinsame Schwenkachse GS schwenkbar. Dies wird dadurch erreicht, daß auf der in Fig. 9 strichliert angedeuteten Achse 17 nicht nur der zweite Schenkel 5 des die Patientenliege 1 tragenden L-förmigen Trägers 2, sondern auch der den C-Bogen 32 aufnehmende Halter 33 in nicht näher dargestellter Weise jeweils in Richtung des gekrümmten Doppelpfeiles "Beta" schwenkbar gelagert ist. Das den C-förmigen Träger 15 mit der Stoßwellenquelle 11 tragende Tragteil 16 ist in nicht näher dargestellter Weise an dem Halter 33 um die gemeinsame Schwenkachse GS in Richtung des gekrümmten Doppelpfeiles "Beta" schwenkbar gelagert. Da die Mittelebene des C-Bogens 32 die gemeinsame Schwenkachse GS enthält und die Mittelachse des C-Bogens 32 die gemeinsame Schwenkachse GS schneidet, ist sichergestellt, daß der Zentralstrahl ZS des Röntgenstrahlenbündels im normalen Betrieb wie erwähnt stets durch den Fokusbereich F der Stoßwellen verläuft.

Infolge der beschriebenen Schwenkbarkeit des C-Bogens 32 mit der Röntgendiagnostikeinrichtung 29 um die durch den Fokusbereich F der Stoßwellen verlaufende gemeinsame Schwenkachse GS sowie infolge der beschriebenen Verstellbarkeit der Röntgendiagnostikeinrichtung 29 mit dem C-Bogen 32 auf einer Kreisbahn um die durch den Fokusbereich F der Stoßwellen verlaufende Mittelachse des C-Bogens 32 ist erreicht, daß nicht nur die Stoßwellenquelle 11, sondern auch Röntgendiagnostikeinrichtung 29 um den ein auf der gemeinsamen Schwenkachse GS liegendes Isozentrum bildenden Fokusbereich F der Stoßwellen sphärisch verstellbar ist. Im folgenden wird daher auch von einer isozentrischen Verstellbarkeit der Röntgendiagnostikeinrichtung 29 gesprochen.

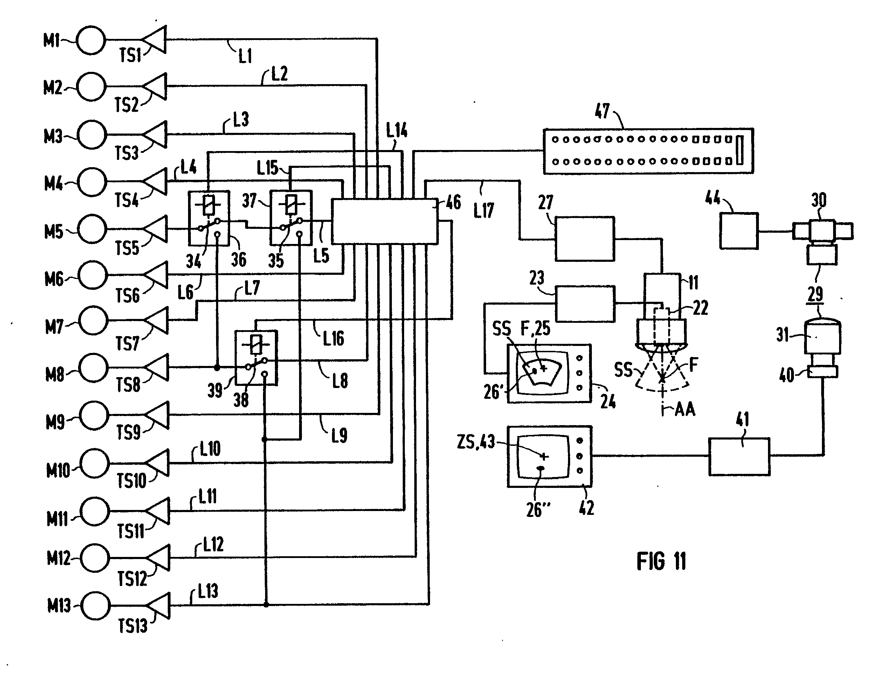

Um die beschriebenen Verstellbewegungen ausführen zu können, sind in den Fig. 8 bis 10 nicht dargestellte Motore M1 bis M13 vorhanden, die unter Zwischenschaltung ebenfalls nicht dargestellter geeigneter Getriebe auf das jeweils zu verstellende Teil einwirken. Die Motore M1 bis M13 sind in Fig. 11 schematisch angedeutet. Dabei dienen die Motore M1 bis M10 den im Zusammenhang mit dem zuvor beschriebenen Ausführungsbeispiel erläuterten Zwecken.

Der Motor M11 dient zur Verstellung des Röntgenbildverstärkers 31 der Röntgendiagnostikeinrichtung 29 in Richtung des Doppelpfeiles w, der Motor 12 bewirkt die Schwenkung der Röntgendiagnostikeinrichtung 29 in Richtung des gekrümmten Doppelpfeiles "Epsilon", und der Motor 13 dient zum Schwenken des Halters 33 mit der Röntgendiagnostikeinrichtung 29 um die gemeinsame Schwenkachse GS in Richtung des gekrümmten Doppelpfeiles "Beta".

Die Motore M1 bis M13 sind unter Zwischenschaltung von schematisch angedeuteten Treiberschaltungen TS1 bis TS13 über Steuerleitungen L1 bis L13 mit einer Steuereinheit 46 verbunden, an die eine Bedieneinheit 47 angeschlossen ist. Die Bedieneinheit 47 besitzt für jeden der Motore M1 bis M13 zwei Tasten, von denen die eine dazu dient, den jeweiligen Motor, solange sie gedrückt ist, in Vorwärtsrichtung laufen zu lassen, und die andere dazu dient, den jeweiligen Motor, solange sie gedrückt ist, in Rückwärtsrichtung laufen zu lassen. Die Motore M5, M8 und M13 und die entsprechenden Getriebe sind so gewählt, daß die Verstellung der Patientenliege 1, der Stoßwellenquelle 11 und der Röntgendiagnostikeinrichtung 29 in Richtung des gekrümmten Doppelpfeiles "Beta" jeweils exakt mit der gleichen Winkelgeschwindigkeit erfolgt. In der von der Steuereinheit 46 zu der zu dem Motor M5 gehörigen Treiberstufe TS5 führenden Steuerleitung L5 liegen in Serie die Umschaltkontakte 34, 35 zweier Relais 36, 37. In der von der Steuereinheit 46 zu der zu dem Motor M8 gehörigen Treiberstufe TS8 führenden Steuerleitung L8 liegt der Umschaltkontakt 38 eines Relais 39. Im Normalfall nehmen die Umschaltkontakte 34, 35 und 38 der Relais 36, 37, 39, deren Erregerspulen über Steuerleitungen L14, L15 und L16 mit der Steuereinheit 46 verbunden sind, die in Fig. 4 gezeigte Schaltstellung ein, so daß die Motore M5, M8 und M13 durch Be tätigen der entsprechenden Tasten der Bedieneinheit 47 unabhängig voneinander aktiviert werden können. Die Bedieneinheit 47 besitzt jedoch vier zusätzliche Tastenpaare, deren Tasten sich von den den Motoren M1 bis M13 zugeordneten Tasten durch ihre andersartige Form unterscheiden. Wird eine der Tasten des ersten zusätzlichen Tastenpaares gedrückt, ändert der Umschaltkontakt 34 des Relais 36, solange die Taste gedrückt ist, seine Schaltstellung, so daß die Treiberstufe TS5 mit der zu der Treiberstufe TS8 führenden Steuerleitung L8 verbunden ist, welche in Abhängigkeit davon, welche der beiden zusätzlichen Tasten gedrückt ist, ein Steuersignal führt, das die Motore M5 und M8 gemeinsam in der einen oder der anderen Richtung rotieren läßt. Solange eine Taste des ersten zusätzlichen Tastenpaares gedrückt ist, werden also die Patientenliege 1 und die Stoßwellenquelle 11 synchron in Richtung des gekrümmten Doppelpfeiles "Beta" um die gemeinsame Schwenkachse GS in der einen oder der anderen Richtung geschwenkt, je nachdem, welche der Tasten des ersten zusätzlichen Tastenpaares aktiviert wird. In entsprechender Weise ändert sich bei Betätigen einer der Tasten des zweiten zusätzlichen Tastenpaares die Schaltstellung des Umschaltkontaktes 35 des Relais 37, so daß die Treiberstufe TS5 mit der zu der Treiberstufe TS13 führenden Steuerleitung L13 verbunden ist, welche in Abhängigkeit davon, welche der beiden zusätzlichen Tasten gedrückt wird, ein Steuersignal führt, das eine gemeinsame Drehung der Motore M5 und M13 in der einen oder der anderen Richtung bewirkt. Je nachdem, welche der beiden Tasten des zweiten zusätzlichen Tastenpaares betätigt wird, werden also die Patientenliege 1 und das Röntgendiagnostikgerät 29 in Richtung des gekrümmten Doppelpfeiles "Beta" in der einen oder anderen Richtung um die gemeinsame Schwenkachse GS geschwenkt. Ebenfalls in entsprechender Weise ändert sich bei Betätigung einer der Tasten des dritten zusätzlichen Tastenpaares die Schaltstellung des Umschaltkontaktes 38 des Relais 39, so daß die Treiberstufe TS8 mit der zu der Treiberstufe TS13 führenden Steuerleitung L13 verbunden ist, welche in Abhängigkeit davon, welche der beiden zusätzlichen Tasten gedrückt ist, ein Steuersignal führt, das eine gemeinsame Rotation der Motore M8 und M13 in der einen oder anderen Richtung veranlaßt. In Abhängigkeit davon, welche der beiden Tasten des dritten zusätzlichen Tastenpaares betätigt wird, werden also die Stoßwellenquelle 11 und die Röntgendiagnostikeinrichtung 29 in der einen oder anderen Richtung in Richtung des gekrümmten Doppelpfeiles "Beta" um die gemeinsame Schwenkachse GS synchron geschwenkt. Wird schließlich eine der Tasten des vierten zusätzlichen Tastenpaares betätigt, ändern sowohl der Umschaltkontakt 35 als auch der Umschaltkontakt 38 ihre jeweilige Schaltstellung, so daß die Treiberstufen TS5 und TS8 mit der zu der Treiberstufe TS13 führenden Steuerleitung L13 verbunden sind, welche in Abhängigkeit davon, welche der beiden zusätzlichen Tasten gedrückt ist, ein Steuersignal führt, das eine gemeinsame Drehung der Motore M5, M8 und M13 in der einen oder anderen Richtung bewirkt. Es erfolgt dann ein gemeinsames synchrones Schwenken der Patientenliege 1, der Stoßwellenquelle 11 und der Röntgendiagnostikeinrichtung 29 in Richtung des gekrümmten Doppelpfeiles "Beta" um die gemeinsame Schwenkachse GS. Es wird also deutlich, daß wahlweise die Patientenliege 1, die Stoßwellenquelle 11 und die Röntgendiagnostikeinrichtung 29 unabhängig voneinander oder zwei der genannten Gerätekomponenten gemeinsam synchron oder aber sämtliche drei Gerätekomponenten gemeinsam synchron um die gemeinsame Schwenkachse GS in der einen oder anderen Richtung in Richtung des gekrümmten Doppelpfeiles "Beta" schwenkbar sind. Dabei ist es aus Gründen, die noch erläutert werden, zweckmäßig, wenn die Stoßwellenquelle 11 und/oder die Röntgendiagnostikeinrichtung 29 gemeinsam mit der Patientenliege 1 -unabhängig von der Stellung, die die Stoßwellenquelle 11 und/ oder die Röntgendiagnostikeinrichtung 29 relativ zu der Patientenliege 1 einnimmt - ausgehend von der in den Fig. 8 bis 10 dargestellten, bezüglich der gemeinsamen Schwenkachse GS horizontalen Lage der Patientenliege 1 wenigstens in einer Richtung um zumindest 45° synchron schwenkbar sind. Im Falle des beschriebenen Ausführungsbeispieles ist in beiden Richtungen eine synchrone Schwenkbarkeit von 90° vorgesehen.

In Fig. 11 sind außerdem wieder die Stoßwellenquelle 11 mit einem in diese integrierten Ultraschall-Sektor-Scanner 22, die Bilderzeugungs- und Steuerelektronik 23 und der Monitor 24 angedeutet. Weiter ist in Fig. 11 wieder die mit der Stoßwellenquelle 11 verbundene Generatorschaltung 27 angedeutet, die über eine Steuerleitung L17 mit der Steuereinheit 46 verbunden. Wird eine sich durch ihre Form und Größe von den übrigen Tasten unterscheidende Taste der Bedieneinheit 47 betätigt, veranlaßt die Steuereinheit 46 die Generatorschaltung 27, die Stoßwellenquelle 11 zur Abgabe einer vorwählbaren Anzahl von aufeinanderfolgenden Stoßwellen anzutreiben.

In Fig. 11 sind auch der Röntgenstrahler 30 und der Röntgenbildverstärker 31 der Röntgendiagnostikeinrichtung 29 schematisch angedeutet. Mit dem Röntgenbildverstärker 31 ist eine schematisch angedeutete Fernsehkamera 40 verbunden, die das Bild des Ausgangsleuchtschirmes des Röntgenbildverstärkers 31 aufnimmt und in ein Videosignal umsetzt, das einer Bildverarbeitungseinrichtung 41 zugeführt wird. Von dieser gelangt das Signal zu einem weiteren Monitor 42, der zur Darstellung des mittels der Röntgendiagnostikeinrichtung 29 gewonnenen Röntgenbildes dient. Mittels der Bildverarbeitungseinrichtung 41 wird in das Monitorbild eine die Lage des Zentralstrahles ZS des Röntgenstrahlenbündels kennzeichnende Marke 43 eingeblendet. Der Röntgenstrahler 30 ist mit einem Röntgengenerator 44 verbunden, der die zum Betrieb des Röntgenstrahlers 30 erforderlichen Spannungen liefert.

Um ein Konkrement 26, z.B. einen Nieren- oder einen Gallenblasenstein, im Körper eines Patienten 12 zu zertrümmern, wird ähnlich wie im Zusammenhang mit dem zuerst beschriebenen Ausführungsbeispiel erläutert vorgegangen. Allerdings wird die Röntgendiagnostikeinrichtung 29, bevor die Stoßwellenquelle 11 in ihre radial innerste Position gebracht wird, so ausgerichtet, daß das Bild 26″ des zu zertrümmernden Konkrementes 26 in dem auf dem Monitor 42 dargestellten Fernsehbild gut sichtbar ist. Anschließend wird der Patient 12 durch Verstellen der Patientenliege 1 in Richtung der Doppelpfeile x und/oder y derart ausgerichtet, daß sich das Bild 26″ des zu zertrümmernden Konkrementes 26 wenigstens in der Nähe der Marke 43 befindet. Erst jetzt wird die Stoßwellenquelle 11 in Richtung des Doppelpfeiles u in ihre radial innerste Position verstellt, in der der Fokusbereich F auf der gemeinsamen Schwenkachse GS und dem Zentralstrahl ZS des Röntgenstrahlenbündels liegt. Dann wird der Patient 12 in der zuvor bereits beschriebenen Weise derart positioniert, daß sich das Bild 26′ des zu zertrümmernden Konkrementes 26 mit der der Position des Fokusbereiches F entsprechenden Marke 25 in dem Bild des Monitors 24 deckt. Ist diese Einstellung gefunden, deckt sich auch das Bild 26″ des zu zertrümmernden Konkrementes 26 auf dem Monitor 42 mit der Marke 43. Ausgehend von der so gefundenen Position besteht nun die Möglichkeit, die Stoßwellenquelle 11 durch Verstellen um den Fokusbereich F als Isozentrum in der gewünschten Weise auszurichten. Erforderlichenfalls kann dabei die Röntgendiagnostikeinrichtung 29 synchron mit der Stoßwellenquelle 11 um die gemeinsame Schwenkachse GS in Richtung des gekrümmten Doppelpfeiles "Beta" geschwenkt werden und/oder die Ausrichtung der Röntgendiagnostikeinrichtung 29 in sonstiger Weise der veränderten Ausrichtung der Stoßwellenquelle 11 angepaßt werden. Dabei besteht infolge des Umstandes, daß nicht nur die Stoßwellenquelle 11, sondern auch die Röntgendiagnostikeinrichtung 29 isozentrisch um den Fokusbereich F verstellt wird, keinerlei Gefahr, daß sich der Fokusbereich F in einer solchen Weise verlagert, daß er außerhalb des zu zertrümmernden Konkrementes 26 zu liegen kommt, bzw. die Röntgendiagnostikeinrichtung 29 eine solche Stellung einnimmt, daß der Zentralstrahl ZS des Röntgenstrahlenbündels nicht durch den Fokusbereich F verläuft. Ist die optimale Ausrichtung der Stoßwellenquelle 11 und der Röntgendiagnostikeinrichtung 29 gefunden, dazu gehört unter Umständen auch, daß die Stoßwellenquelle 11 und die Röntgendiagnostikeinrichtung 29 relativ zueinander derart ausgerichtet sind, daß eine Abbildung der Stoßwellenquelle 11 in dem Röntgenbild im wesentlichen unterbleibt, kann mit der Stoßwellenbehandlung begonnen werden. Falle dies erforderlich ist, kann vor oder während der Behandlung ein synchrones Schwenken der Patientenliege mit der Stoßwellenquelle 11 und/oder der Röntgendiagnostikeinrichtung 29 um die gemeinsame Schwenkachse GS in Richtung des gekrümmten Doppelpfeiles "Beta" erfolgen, um die Lage eines zu zertrümmernden Konkrementes durch Schwerkraftwirkung zu stabilisieren. Auch dabei treten keinerlei Verlagerungen des Fokusbereiches F auf. Ebenso ist stets gewährleistet, daß der Zentralstrahl ZS des Röntgenstrahlenbündels durch den Fokusbereich F verläuft. Es kann allenfalls erforderlich sein, die Ausrichtung des Patienten relativ zu der Stoßwellenquelle 11 durch Verstellen der Patientenliege 1 geringfügig zu korrigieren, um einer eventuellen Veränderung der Lage des Konkrementes 26 Rechnung zu tragen.

Auch bei dem Gerät gemäß den Fig. 8 bis 11 besteht die Möglichkeit, den Stoßwellengenerator 11 in eine Untertischposition bzw. eine Parkposition zu bringen, und zwar in der gleichen Weise, wie dies zuvor unter Bezugnahme auf die Fig. 5 bis 7 beschrieben wurde. Außerdem kann es für bestimmte Behandlungsfälle zweckmäßig sein, abweichend von den Fig. 8 bis 10 den Röntgenstrahler 30 in Unter- und den Röntgenbildverstärker 31 in Obertischposition zu bringen. Hierzu wird der C-Bogen 32 um die gemeinsame Schwenkachse GS ausgehend von der in den Fig. 8 bis 10 gezeigten Position um 180° geschwenkt. Bezogen auf die Fig. 8 erfolgt die Schwenkbewegung des C-Bogens 32 um die gemeinsame Schwenkachse GS gegen den Uhrzeigersinn. Um dabei zu vermeiden, daß Bestandteile der Röntgendiagnostikeinrichtung 29 mit der Patientenliege 1 kollidieren, wird diese, wieder bezogen auf Fig. 8 in Richtung des Doppelpfeiles x in ihre äußerste rechte Position verstellt und zusätzlich der C-Bogen 32 in Richtung des gekrümmten Doppelpfeiles "Epsilon" derart verstellt, daß sich der Röntgenstrahler 30 so nahe als möglich bei dem Halter 33 befindet. Es ist dann möglich, den C-Bogen 32 an dem in Fig. 8 linken Ende der Patientenliege 1 vorbeizuschwenken, ohne daß Kollisionen auftreten. Zuvor wird der Wagen 7 in eine solche Höhe gebracht, daß die zum Schwenken des C-Bogens 32 erforderliche Bodenfreiheit vorliegt.

Die Radien des C-förmigen Trägers 15 und des C-Bogens 32 weichen übrigens um ein solches Maß voneinander ab, daß der C-Bogen 32 über den C-förmigen Träger 15 um die gemeinsame Schwenkachse GS zumindest dann hinweg geschwenkt werden kann, wenn die Stoßwellenquelle 11 in Richtung des Doppelpfeiles u in ihre radial innerste und der Röntgenbildverstärker 31 in Richtung des Doppelpfeiles w in seine radial äußerste Position verstellt ist.

Infolge des Umstandes, daß die Mittelebene des die Stoßwellenquelle 11 tragenden C-förmigen Trägers 15 im Abstand von der gemeinsamen Schwenkachse GS verläuft und die Mittelebene des C-Bogens 32 der Röntgendiagnostikeinrichtung 29 die gemeinsame Schwenkachse GS enthält, wird sich in der Regel eine dem jeweiligen Behandlungsfall abgepaßte Ausrichtung der Röntgendiagnostikeinrichtung 29 und der Stoßwellenquelle 11 finden lassen, in der sich der C-förmige Träger 15 außerhalb des Strahlenganges der Röntgendiagnostikeinrichtung 29 befindet.

Im Falle des beschriebenen Ausführungsbeispieles sind die Stoßwellenquelle 11 und die Röntgendiagnostikeinrichtung 29 um den Fokusbereich F als auf der gemeinsamen Schwenkachse GS liegendes Isozentrum sphärisch verstellbar. Das beschriebene synchrone Schwenken von der Stoßwellenquelle 11 und/oder der Röntgendiagnostikeinrichtung 29 mit der Patientenliege 1 um die gemeinsame Schwenkachse GS ist ohne Verlagerungen des Fokusbereiches F bzw. des Zentralstrahles ZS jedoch auch dann möglich, wenn keine isozentrische, sondern eine andere Verstellbarkeit der Stoßwellenquelle 11 und der Röntgendiagnostikeinrichtung 29 relativ zu der Patientenliege 1 gegeben ist und der Fokusbereich F nicht auf der gemeinsamen Schwenkachse GS liegt bzw. der Zentralstrahl ZS die gemeinsame Schwenkachse GS nicht schneidet. Es sind auch Ausführungsformen möglich, bei denen eine isozentrische Verstellbarkeit nur der Stoßwellenquelle 11 oder nur der Röntgendiagnostikeinrichtung 29 vorgesehen ist.

Auch die Bilderzeugungs- und Steuerelektronik 23 und gegebenenfalls der Röntgengenerator 44 sind in nicht dargestellter Weise mit der Steuereinheit 18 bzw. 46 verbunden, wobei die Bedieneinheit 19 bzw. 47 über nicht dargestellte zusätzliche Bedienelemente verfügt, die der Elektronik 23 und gegebenenfalls dem Generator 44 zugeordnet sind.

Im Falle beider Ausführungsbeispiele ist die Zertrümmerung von Konkrementen beschrieben. Es versteht sich, daß das erfindungsgemäße Gerät auch zur Behandlung anderer Leiden verwendet werden kann.

## Ansprüche

1. Gerät zur Behandlung eines Lebewesens (12) mit fokussierten Stoßwellen, welches als Gerätekomponenten eine Patientenliege (1) und eine Stoßwellenquelle (11) aufweist, die relativ zu der Patientenliege (1) verstellbar ist, die Mittel (13) zur akustischen Koppelung mit dem Körper des Lebewesens (12) aufweist und mittels derer in einem auf der akustischen Achse (AA) der Stoßwellenquelle (11) liegenden Fokusbereich (F) zusammenlaufende Stoßwellen erzeugbar sind, **dadurch gekennzeichnet,** daß eine gemeinsame Schwenkachse (GS) vorgesehen ist, um die die Gerätekomponenten wahlweise unabhängig voneinander oder synchron schwenkbar sind.

2. Gerät nach Anspruch 1, **dadurch gekennzeichnet,** daß als weitere Gerätekomponente eine Ortungseinrichtung (29) vorgesehen ist, die relativ zu den anderen Gerätekomponenten verstellbar ist, die zur Ortung eines therapeutisch relevanten Bereiches (26) dient und die wahlweise unabhängig von den anderen Gerätekomponenten oder synchron mit wenigstens einer der anderen Gerätekomponenten um die gemeinsame Schwenkachse (GS) schwenkbar ist.

3. Gerät nach Anspruch 1 oder 2, **dadurch gekennzeichnet,** daß die gemeinsame Schwenkachse (GS) im wesentlichen horizontal und/oder etwa quer zur Längsachse der Patientenliege (1) verläuft.

4. Gerät nach einem der Ansprüche 1 bis 3, **dadurch gekennzeichnet,** daß die Patientenliege (1) und wenigstens eine andere Gerätekomponente ausgehend von einer waagerechten Lage der Patientenliege (1) unabhängig von der Stellung der wenigstens einen anderen Gerätekomponente relativ zu der Patientenliege (1) in wenigstens einer Richtung um zumindest 45° synchron schwenkbar sind.

5. Gerät nach einem der Ansprüche 1 bis 4, **dadurch gekennzeichnet,** daß unabhängig von der Stellung der Patientenliege (1), die diese bezüglich der gemeinsamen Schwenkachse (GS) einnimmt, zumindest eine andere Gerätekomponente um wenigstens 180° relativ zu der Patientenliege (1) schwenkbar ist.

6. Gerät nach einem der Ansprüche 2 bis 5, **dadurch gekennzeichnet,** daß die Ortungseinrichtung (29) unabhängig von den beiden anderen Gerätekomponenten um ein Isozentrum sphärisch verstellbar ist und daß der mittels der Ortungseinrichtung (29) ortbare therapeutisch relevante Bereich (26) das Isozentrum enthält.

7. Gerät nach einem der Ansprüche 1 bis 6, **dadurch gekennzeichnet,** daß die Stoßwellenquelle (11) unabhängig von der Patientenliege (1) und gegebenenfalls der Ortungseinrichtung (29) um ein Isozentrum sphärisch verstellbar ist und daß der Fokusbereich (F) der Stoßwellen das Isozentrum enthält.

8. Gerät nach den Ansprüchen 6 und 7, **dadurch gekennzeichnet,** daß die Stoßwellenquelle (11) und die Ortungseinrichtung (29) um das gleiche Isozentrum schwenkbar sind.

9. Gerät nach einem der Ansprüche 6 bis 8, **dadurch gekennzeichnet,** daß die gemeinsame Schwenkachse (GS) durch das Isozentrum verläuft.

10. Gerät nach einem der Ansprüche 6 bis 9, **dadurch gekennzeichnet,** daß für die Ortungseinrichtung (29) ein um die gemeinsame Schwenkachse (GS) schwenkbarer C-förmiger Träger (32) vorgesehen ist, dessen Mittelachse parallel zu der gemeinsamen Schwenkachse (GS) verläuft und an dem die Ortungseinrichtung (29) derart angebracht ist, daß sie auf einer Kreisbahn um das Isozentrum verstellbar ist.

11. Gerät nach einem der Ansprüche 7 bis 10, **dadurch gekennzeichnet,** daß für die Stoßwellenquelle (11) ein um die gemeinsame Schwenkachse (GS) schwenkbarer C-förmiger Träger (15) vorgesehen ist, dessen Mittelachse parallel zu der gemeinsamen Schwenkachse (GS) verläuft und an dem die Stoßwellenquelle (11) derart angebracht ist, daß sie auf einer Kreisbahn um das Isozentrum verstellbar ist.

12. Gerät nach Anspruch 11, **dadurch gekennzeichnet,** daß die Mittelebene des die Stoßwellenquelle (11) tragenden C-förmigen Trägers (15) im Abstand von der gemeinsamen Schwenkachse (GS) verläuft und die Stoßwellenquelle (11) derart an dem C-förmigen Träger (15) angebracht ist, daß ihre akustische Achse (AA) parallel zur Mittelebene des C-förmigen Trägers (15) verläuft und die gemeinsame Schwenkachse (GS) schneidet.

13. Gerät nach Anspruch 10 und Anspruch 11 oder 12, **dadurch gekennzeichnet,** daß die Radien der für die Ortungseinrichtung (29) und die Stoßwellenquelle (11) vorgesehenen C-förmigen Träger (15, 32) derart unterschiedlich gewählt sind, daß die Ortungseinrichtung (29) und die Stoßwellenquelle (11) kollisionsfrei übereinander hinweg um die gemeinsame Achse (GS) schwenkbar sind.

14. Gerät nach Anspruch 10 und Anspruch 11 oder 12 oder nach Anspruch 13, **dadurch gekennzeichnet,** daß die Mittelebenen der C-förmigen Träger (15, 32) derart unterschiedliche Abstände von der gemeinsamen Schwenkachse (GS) aufweisen, daß bei parallel zueinander ausgerichteten C-förmigen Trägern (15, 32) ein Abstand zwischen den einander zugewandten Flächen der C-förmigen Träger (15, 32) vorliegt.

15. Gerät nach einem der Ansprüche 11 bis 14, **dadurch gekennzeichnet,** daß der die Stoßwellenquelle (11) tragende C-förmige Träger (15) mit der Stoßwellenquelle (11) in eine Parkstellung schwenkbar ist, in der die Mittel ebene des C-förmigen Trägers (15) im wesentlichen rechtwinklig zur gemeinsamen Schwenkachse (GS) verläuft.

16. Gerät nach einem der Ansprüche 10 bis 15, **dadurch gekennzeichnet,** daß als Ortungseinrichtung ein einen Röntgenstrahler (30) und einen diesem gegenüberliegenden Strahlenempfänger (31) aufweisendes Röntgendiagnostikgerät (29) vorgesehen ist, wobei mittels des Röntgenstrahlers (30) ein Röntgenstrahlenbündel erzeugbar ist, dessen Zentralstrahl (ZS) durch das Isozentrum zu dem Strahlenempfänger (31) verläuft, und daß der die Röntgendiagnostikeinrichtung (29) tragende C-förmige Träger als C-Bogen (32) ausgeführt ist, an dessen Enden der Röntgenstrahler (30) und der Strahlenempfänger (31) angebracht sind, wobei der C-Bogen (32) in einem Halter (33) derart längs seines Umfanges verschiebbar aufgenommen ist, daß die Röntgendiagnostikeinrichtung (29) um das Isozentrum schwenkbar ist, und wobei der C-Bogen (32) um die gemeinsame Schwenkachse (GS) schwenkbar ist.

17. Gerät nach Anspruch 16, **dadurch gekennzeichnet,** daß die Mittelebene des C-Bogens (32) die gemeinsame Schwenkachse (GS) enthält und daß die Mittelebene des die Stoßwellenquelle (11) tragenden C-förmigen Trägers (15) im Abstand von der gemeinsamen Schwenkachse (GS) verläuft und die Stoßwellenquelle (11) derart an dem C-förmigen Träger (15) angebracht ist, daß ihre akustische Achse (AA) parallel zu der Mittelebene des C-förmigen Trägers (15) verläuft und den Zentralstrahl (ZS) des Röntgenstrahlenbündels in der gemeinsamen Schwenkachse (GS) schneidet.

18. Gerät nach einem der Ansprüche 1 bis 17, **dadurch gekennzeichnet,** daß die Patientenliege (1) an einem L-förmigen Träger (2) angebracht ist, der mit seinem einen Ende um die gemeinsame Schwenkachse (GS) schwenkbar gelagert ist und an dessen anderem Ende die Patientenliege (1) freitragend befestigt ist.

19. Gerät nach einem der Ansprüche 1 bis 18, **dadurch gekennzeichnet,** daß die Stoßwellenquelle (11) Ultraschall-Ortungsmittel (22) zur Ortung eines therapeutisch relevanten Bereiches (26) enthält.

20. Verwendung eines Gerätes nach einem der Ansprüche 1 bis 19 zur berührungslosen Zertrümmerung von im Körper eines Lebewesens (12) befindlichen Konkrementen (26).

FIG 1

EP 0 405 282 A1

FIG 2

FIG 3

EP 0 405 282 A1

M1 TS1 S1

M2 TS2 S2

M3 TS3 S3

M4 TS4 S4 S11

M5 TS5 S5 21 20

M6 TS6 S6

M7 TS7 S7

M8 TS8 S8

M9 TS9 S9

M10 TS10 S10

18

19

S12

27

23

11

22

F,25

24

26'

SS

SS

F

AA

**FIG 4**

EP 0 405 282 A1

FIG 7

FIG 5

FIG 6

20

EP 0 405 282 A1

FIG 8

FIG 9

EP 0 405 282 A1

FIG 10

FIG 11

EP 0 405 282 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 286 170 (PHILIPS) <br> * Ansprüche 1,2; Figuren 1,2 * <br> --- | 1-3 | A 61 B 17/22 |
| A | DE-U-8 800 985 (DORNIER) <br> * Seite 3, Zeile 7 - Seite 4, Zeile 18; Figur 3 * <br> ----- | 1-3 | |
| | | | **RECHERCHIERTE SACHGEBIETE (Int. Cl.5)** |
| | | | A 61 B |

Der vorliegende Recherchenbericht wurde für alle Patentansprüche erstellt

| Recherchenort | Abschlußdatum der Recherche | Prüfer |
|---|---|---|
| DEN HAAG | 01-10-1990 | MOERS R.J. |